# EUROPEAN PATENT APPLICATION

(11) **EP 4 825 545 A1**
(43) Date of publication of application: **21.10.2026**
(21) Application number: 24902704.6
(22) Date of filing: 05.12.2024
(51) Int. Cl.: B25J 17/00, B25J 9/16, A61B 34/30

(54) **MULTI-DEGREE-OF-FREEDOM ROBOT AND CONTROL METHOD THEREFOR**

(30) Priority: 14.12.2023 CN 202323428221 U
(71) Applicant: Guangzhou DeepSurgery Medical Instrument Technology Co., Ltd, Guangzhou, Guangdong 510000 (CN)
(72) Inventor: ZHU, Qingtang, Hong Kong Smart Valley Yuexiu iPARK Qingsheng Hub Pilot Launch Zone Nansha District Guangzhou, Guangdong 510000 (CN); WANG, Chuyu, Hong Kong Smart Valley Yuexiu iPARK Qingsheng Hub Pilot Launch Zone Nansha District Guangzhou, Guangdong 510000 (CN); HUANG, Xijun, Hong Kong Smart Valley Yuexiu iPARK Qingsheng Hub Pilot Launch Zone Nansha District Guangzhou, Guangdong 510000 (CN); FAN, Jingyuan, Hong Kong Smart Valley Yuexiu iPARK Qingsheng Hub Pilot Launch Zone Nansha District Guangzhou, Guangdong 510000 (CN)
(74) Representative: Petraz, Gilberto Luigi
(86) International application number: PCT/CN2024/137199
(87) International publication number: WO 2025/124283

(57) **Abstract**

The present invention relates to a multi-degree-of-freedom robot, including a first joint, a second joint, a third joint, and an end-effector assembly that are connected sequentially. The first joint, the second joint, the third joint, and the end-effector assembly each include at least one linear motor and an installing plate for installing the linear motor. At least one of the first joint, the second joint, and the third joint further includes a rotating motor. The three joints of the robot can not only implement linear motion in three axis directions, but also implement rotational motion in at least one of the directions, which ultimately enables the end-effector assembly to move more flexibly and reach a lesion position more easily.

## Description

### TECHNICAL FIELD

The present invention relates to the field of medical robots, and in particular to a multi-degree-of-freedom robot and a control method therefor.

### BACKGROUND

With the development of the automation technology, various automation auxiliary devices such as robots are applied to all walks of life. For example, in the medical industry, various auxiliary devices including surgical robots are provided, such as abdominal surgery robots, orthopedic surgical robots, and the like.

Existing surgical robots each are provided with a plurality of joints, usually with more than three joints, to ensure that the surgical robots can drive end-effectors to perform flexible motion during operation. However, the joints of the surgical robot in current stage are usually in the form of linear motors or rotating motors. A linear motor allows different joints to perform linear motion in three directions which are an X-axis direction, a Y-axis direction, and a Z-axis direction to control the motion of the end-effectors, and a rotating motor allows the joints to perform rotational motion in the X-axis direction, the Y-axis direction, and the Z-axis direction to control the motion of the end-effectors. No matter which of the above forms, a degree of freedom for the motion of the robot is still limited, and the flexibility of the surgical robot still needs to be improved.

With the development of the automation technology, various automation auxiliary devices such as robots are applied to all walks of life. For example, in the medical industry, various auxiliary devices including surgical robots are provided, such as abdominal surgery robots, orthopedic surgical robots, and the like.

Existing surgical robots each are provided with a plurality of joints, usually with more than six joints, to ensure that the surgical robots can drive the end-effectors to perform flexible motion during operation. The surgical robot is usually designed with a structure that is provided with a plurality of rotating motors in series, to provide linear spatial freedom at an end, while a rope-driven, wire-driven, or hinge-driven wrist-like structure is used on the end-effector to provide flexible rotation and swing angle for a tool. However, the robot based on the above design has some drawbacks in the application to microsurgery. First, loads are accumulated in the plurality of rotating motors in series, so that the overall robotic arms are designed relatively large, which cannot meet narrow working space for the microsurgery in a microscope well. Second, the rope-driven, wire-driven, or hinge-driven wrist-like structure is used to provide the flexible swing angle for the tool, which may not provide reliable guaranty in terms of driving accuracy, and a size (usually less than 3 mm, with an end reaching 0.05-0.1 mm) of an instrument for the microsurgery is much smaller than a size of an instrument for general surgery. Therefore, it is difficult to produce a rope-driven, wire-driven, or hinge-driven wrist-like structure with an ultra-small size. Last but not least, there are also some miniaturized robots such as ophthalmic surgical robots that are designed with parallelogram or linear motors connected in parallel. Although these mechanisms can meet the needs of microsurgery in terms of size and precision, these mechanisms cannot provide flexible degrees of freedom at ends due to the lack of wrist-like structures to provide the flexible swing angles for the tools, which cannot meet the needs of complex actions such as microsurgical suture and knotting.

### SUMMARY

The present invention provides a multi-degree-of-freedom robot to enable an end-effector to perform linear motion and rotary motion simultaneously in three axis directions, to overcome the above problem of poor flexibility of a surgical robot in the prior art.

To resolve the above technical problem, technical solutions used in the present invention are as follows: a multi-degree-of-freedom robot, including a first joint, a second joint, a third joint, and an end-effector assembly that are connected sequentially. The first joint, the second joint, the third joint, and the end-effector assembly each include at least a linear motor and an installing plate for installing the linear motor; and at least one of the first joint, the second joint, and the third joint further includes a rotating motor.

In the above technical solution, each joint is provided with a linear motor. When the linear moving directions of all linear motors is set to be different from each other and perpendicular to each other, linear motion in the three axis directions can be implemented. The rotating motor can further enable at least one joint to rotate, improving the flexibility of the joints of the robot. In addition, moving directions of the linear motor may also be the same, which can increase a range of movement of the robot in one of the directions.

Preferably, a damper is installed on the installing plate corresponding to a linear motor whose output axis is parallel to a gravitational direction of the end-effector assembly, and an output end of the damper is connected to an output end of the linear motor. More preferably, the damper is a coil spring. For a linear motor whose output axis is parallel to a gravitational direction of the end-effector assembly, there is a large gravitational interference as the linear motor is affected by the gravity of the end-effector assembly. In addition, if the linear motor is located at the third joint, the joint is equivalent to an elongated connecting rod between the first rotating motor and the second rotating motor with loads thereof, and bears a large inertial torque. Therefore, during use, to prevent joint tremor, positioning accuracy decrease, or spoiled motor lifespan, an elastic force direction of the damper, that is, the elastic force direction of the coil spring is disposed opposite to the gravitational direction of end-effector assembly. When this linear motor moves, the coil spring can provide constant force output to balance a vertical load that the third linear motor bears. Benefited from following of a leaf spring of the coil spring, the design can ensure that an output elastic force of gravity compensation is in a horizontal direction of the third linear motor, to prevent the deformation and damage of the coil spring caused by the change in a direction of the compensating elastic force, and a force applied to the linear motor in another direction is prevented. It should be pointed out that an output force K of a constant-force clockwork spring is preset to be 2N, which is calculated by a rated thrust T of the motor, an expected maximum operating applied force A, and a born load gravity G, which can be properly adjusted based on the above parameters, and a value range of K is calculated as follows: $\left\{\begin{matrix}T + G - A \geq K \geq A + G - T ; \\ G \geq K > 0\end{matrix}\right. .$

Preferably, the first joint includes a first installing plate and a first linear motor connected to the first installing plate; the second joint includes a second installing plate connected to an output end of the first linear motor, a second linear motor connected to the second installing plate, a first installing component connected to an output end of the second linear motor, and a first rotating motor installed on the first installing component; and the third joint includes a third installing plate connected to an output end of the first rotating motor, a third linear motor connected to the third installing plate, a second installing component connected to an output end of the third linear motor, and a second rotating motor connected to the second installing component; and a connecting component is connected to an output end of the first rotating motor. An output axis of the second linear motor is perpendicular to an output axis of the first linear motor, and an output axis of the third linear motor is perpendicular to both the output axis of the second linear motor and the output axis of the first linear motor; a rotating axis of the first rotating motor is perpendicular to the output axis of the second linear motor, a rotating axis of the second rotating motor is perpendicular to the output axis of the third linear motor, and the output axis of the third linear motor is parallel to a gravitational direction of the end-effector assembly. The first linear motor, the second linear motor, and the third linear motor respectively enable an actuator mechanism to translate in three directions which are an X-axis direction, a Y-axis direction, and a Z-axis direction, and the first rotating motor and the second rotating motor provide rotational degrees of freedom in two directions based on translation in the three directions, so that a motion joint of the robot can move more flexibly. The third linear motor is a linear motor with an output axis parallel to the gravitational direction of the end-effector assembly, and a load of the third linear motor is the second rotating motor and the end-effector assembly, so that a less load is provided for the third linear motor. If the third linear motor is changed to the first linear motor or the second linear motor, more load is provided, and a load of the second joint and/or a load of the third joint also need to be assumed.

Preferably, the end-effector assembly includes a connecting component installed at the output end of the second rotating motor, a fourth linear motor installed on the connecting component, an end installing base installed at an output end of the fourth linear motor, and an actuator mechanism installed on the end installing base; and the actuator mechanism includes an actuating instrument, an end motor base installed on the end installing base, and an instrument rotating motor installed at the bottom of the end motor base; and the actuating instrument is connected to an output end of the instrument rotating motor. The fourth linear motor may be a linear motor, specifically a fourth linear motor.

Preferably, a counterweight block is disposed on one side of the third installing plate away from the third linear motor, and a distance between the counterweight block and an axis of the first rotating motor is *Dl*/2, specifically: $Dl = d \sqrt{1 + {\left(\left(l4+l0\right)cos\left(R2\right)/d\right)}^{2}}$ where *Dl* is a change value of a force arm applied to the first rotating motor; *l*4 is a displacement of the fourth linear motor; *l*0 is a displacement of the instrument linear motor; *R*2 is a rotation angle of the second rotating motor; and *d* is an initial value of the force arm applied to the first rotating motor.

A rotating axis of the first rotating motor and a rotating axis of the second rotating motor are disposed to be orthogonal to a central axis of an end instrument at one point, and the second rotating motor, the third linear motor, the fourth linear motor, the instrument rotating motor, and the instrument linear motor are main loads of the first rotating motor, and centers of gravity of the loads are away from the first rotating motor. In addition, the first rotating motor is a main attitude rotation mechanism, and is subjected to a large load gravitational torque. With self-rotation, and subsequent dynamic motion of the second rotating motor, the third linear motor, the fourth linear motor, the instrument rotating motor, and the instrument linear motor, the effect of the torque on the first rotating motor is relatively unstable. Under the action of the counterweight block, a force arm applied to the first rotating motor can be reduced by 2/3 cm, so that the stability of an overall structure and the smoothness of dynamic operation are ensured.

Preferably, the third installing plate is L-shaped, a first side plate of the third installing plate is connected to the output end of the first rotating motor, and a second side plate is configured to install the third linear motor; and the counterweight block is installed at one end of the first side plate away from the second side plate.

Preferably, the actuator mechanism includes an end motor base installed on the end installing base and an instrument rotating motor installed at the bottom of the end motor base; and the actuating instrument is connected to an output end of the instrument rotating motor. The instrument rotating motor drives the actuating instrument to perform self-rotation, and rotation of the actuating instrument does not cause an end of the actuating instrument to produce a relative displacement with a camera module. Therefore, the accuracy of an image collected by the camera module can still be ensured. The instrument rotating motor can enable the actuating instrument to move more flexibly, and to facilitate the alignment of the actuating instrument with a tissue lesion.

Preferably, the instrument rotating motor is a hollow motor; the actuator mechanism further includes an instrument linear motor installed on the end motor base and a push rod installed at an output end of the instrument linear motor; the push rod passes through the end motor base and the hollow motor and extends into the actuating instrument, and is configured to promote opening and closing of the actuating instrument. When the actuating instrument is a surgical instrument such as microforceps or microscissors, in order to implement that the instrument is remotely controlled to be opened and closed, the push rod is driven into the actuating instrument through the instrument linear motor to promote its opening or closing.

Preferably, the instrument rotating motor is connected to the actuating instrument through an instrument installing base; and the actuating instrument is detachably connected to the instrument installing base. Because the actuating instrument needs to be replaced frequently, if the actuating instrument is directly connected to the instrument rotating motor, an output end of the instrument rotating motor needs to be operated every time the actuating instrument is detached, with service life easily affected. In addition, a fastener is not easily disassembled and assembled, and especially necessary disassembly and assembly tools are not provided in an operating room environment. After the instrument installing base is added, the instrument installing base can be connected to an output end of the instrument rotating motor through a fastener, and the actuating instrument only needs to be connected to the instrument installing base through snap connection or threaded connection, so that the actuating instrument is replaced more easily, and detachment performed on the instrument rotating motor is not required.

Preferably, a camera module is further installed on the end installing base, an imaging axis of the camera module is parallel to an axis of the actuating instrument, and a focus of the camera module is located on a plane perpendicular to an end of the actuating instrument. Preferably, because the camera module and the actuator assembly are both installed on the end installing base, the camera module and the actuator assembly are always in a state of moving together, so that the robot can have dynamic vision and keep a state of focusing. When the actuating instrument moves in any direction, the camera module can collect an image in the direction that the actuating instrument is in. In addition, because the actuator assembly and a motion model are separately installed on the end installing base, the actuator assembly and the motion model do not interfere with each other, and the camera module does not block the movement of the actuator assembly. Because the camera module and the end of the actuating instrument are in a relatively stationary state, the camera module can always be in the state of focusing the end of the actuating instrument, so that the camera module can take a clear image of the end of the actuating instrument no matter when the actuating instrument performs any movement.

Preferably, two first linear motors are disposed side by side on the first installing plates. Because a load of the first linear motor is maximum, a sufficient thrust can be generated at the first joint through the two first linear motors.

Preferably, motion directions of the first linear motor, the second linear motor, and the third linear motor are orthogonal to each other; the first linear motor and the second linear motor are all orthogonal to a rotating axis of the first rotating motor; a motion axis of the third linear motor is parallel to a rotating axis of the first rotating motor; the motion axis of the third linear motor and the motion axis of the fourth linear motor are orthogonal to a rotating axis of the second rotating motor; and rotating axes of the first rotating motor, the second rotating motor, and the instrument rotating motor intersect at one point, and the point is located on an axis of the actuating instrument. Driving of the first linear motor, second linear motor, third linear motor, fourth linear motor, as well as the first rotating motor, second rotating motor, and the instrument rotating motor in the x, y, z, and tool axis directions, at the roll angle, at the pitch angle, and at the yaw angle, totaling seven degrees of freedom, are all transmitted to the axis point O of the end-tool without any theoretical loss.

Compared with the prior art, beneficial effects of the present invention are as follows: the three joints of the robot can not only implement linear motion in the three axis directions, but also implement rotational motion in at least one of the directions, and such motion is transmitted from a corresponding drive motor to a tool axis without damage through effective structural design, and the end-effector assembly moves flexibly and reaches a lesion position more easily.

(2) In addition, benefited from a special combination of the linear motor and the rotating motor, the mechanism can be driven in a variety of ways, implementing three-axis translation and virtual fixed point control in a plurality of planes, ultimately enabling the end-effector assembly to implement higher precision and compact virtual wrist-like joint motion than in a rope-driven, wire-driven, or hinge-driven manner.

### BRIEF DESCRIPTION OF DRAWINGS

FIG. 1 is a three-dimensional view of a multi-degree-of-freedom robot according to the present invention;
FIG. 2 is an exploded view of a first joint, a second joint, and a third joint according to the present invention;
FIG. 3 is a schematic structural diagram of the third joint according to the present invention;
FIG. 4 is an exploded view of an end-effector assembly according to the present invention;
FIG. 5 is a position relationship diagram of a first linear motor, a second linear motor, a third linear motor, a fourth linear motor, a first rotating motor, a second rotating motor, and an instrument rotating motor of the multi-degree-of-freedom robot according to the present invention;
FIG. 6 is a schematic diagram of a first virtual wrist-like joint according to the present invention;
FIG. 7 is a schematic diagram of a second virtual wrist-like joint according to the present invention;
FIG. 8 is another schematic diagram of the first virtual wrist-like joint according to the present invention; and
FIG. 9 is another schematic diagram of the second virtual wrist-like joint according to the present invention.

### DETAILED DESCRIPTION OF EMBODIMENTS

The accompanying drawings are for illustrative purposes only, and should not be construed as limiting the patent. To better describe the embodiments, some components of the accompanying drawings are omitted, enlarged, or reduced, and do not represent actual product sizes. For those skilled in the art, some commonly known structures and descriptions thereof in the drawings can be understood. The description of the positional relationship in the accompanying drawings is for illustrative purposes only and cannot be construed as a limitation on the patent.

Same or similar numerals in the drawings of embodiments of the present invention are corresponding to same or similar components. In the descriptions of the present invention, it should be understood that an orientation or a position relationship indicated by a term "up", "down", "left", "right", "long", "short" or the like is based on an orientation or a position relationship shown in the accompanying drawings, and is merely intended for ease of describing the present invention and simplifying description, but does not indicate or imply that a described apparatus or element needs to have a specific orientation or be constructed and operated in a specific orientation. Therefore, the terms used in the drawings describing the positional relationship are only for illustrative purposes and cannot be understood as limiting the patent. Those skilled in the art may understand specific meanings of the above terms according to specific cases.

Technical solutions of the present invention are further described in detail below through specific embodiments and with reference to accompanying drawings.

### Embodiment 1

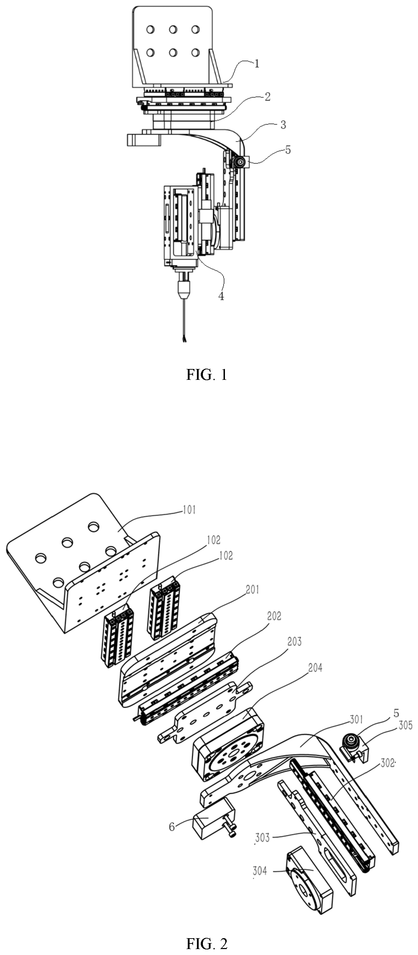

FIG. 1 to FIG. 3 show a multi-degree-of-freedom robot in embodiment 1, including a first joint 1, a second joint 2, a third joint 3, and an end-effector assembly 4 that are connected sequentially. The first joint 1, the second joint 2, the third joint 3, and the end-effector assembly 4 each include at least one linear motor and an installing plate for installing the linear motor. At least one of the first joint 1, the second joint 2, and the third joint 3 further includes a rotating motor. A damper 5 is installed on the installing plate corresponding to a linear motor whose output axis is parallel to a gravitational direction of the end-effector assembly 4, and an output end of the damper 5 is connected to an output end of the linear motor. In this embodiment, the damper 5 is a coil spring.

Specifically, the first joint 1 includes a first installing plate 101 and a first linear motor 102 connected to the first installing plate 101. In this embodiment, two first linear motors 102 are arranged side by side. The second joint 2 includes a second installing plate 201 connected to an output end of the first linear motor 102, a second linear motor 202 connected to the second installing plate 201, a first installing component 203 connected to an output end of the second linear motor 202, and a first rotating motor 204 installed on the first installing component 203. The third joint 3 includes a third installing plate 301 connected to an output end of the first rotating motor 204, a third linear motor 302 connected to the third installing plate 301, a second installing component 303 connected to an output end of the third linear motor 302, and a second rotating motor 304 connected to the second installing component 303. A connecting component 401 is connected to an output end of the second rotating motor 304. An output axis of the second linear motor 202 is perpendicular to an output axis of the first linear motor 102, and an output axis of the third linear motor 302 is perpendicular to both the output axis of the second linear motor 202 and the output axis of the first linear motor 102. A rotating axis of the first rotating motor 204 is perpendicular to the output axis of the second linear motor 202, a rotating axis of the second rotating motor 304 is perpendicular to the output axis of the third linear motor 302, and the output axis of the third linear motor 302 is parallel to a gravitational direction of the end-effector assembly 4. The first linear motor 102, the second linear motor 202, and the third linear motor 302 respectively enable an actuator mechanism to translate in three directions which are an X-axis, a Y-axis, and a Z-axis, and the first rotating motor 204 and the second rotating motor 304 provide rotational degrees of freedom in two directions based on translation in the three directions, so that a motion joint of the robot can move more flexibly. In this embodiment, the third linear motor 302 is a linear motor with an output axis parallel to the gravitational direction of the end -effector assembly 4. The coil spring is firmly installed on the third installing plate 301 by a fastening base 305 and is located on one side close to the first rotating motor 204, and a movable end of the coil spring is connected to an output end of the third linear motor 302. In this embodiment, the movable end of the coil spring is firmly connected to the second installing component 303, and the third linear motor is affected by the gravity of the end-effector assembly 4, so that there is a large gravitational interference. In addition, if the linear motor is located at the third joint 3, the joint is equivalent to an elongated connecting rod between the first rotating motor 204 and the second rotating motor 304 with loads thereof, and bears a large inertial torque. Therefore, during use, to prevent joint tremor, positioning accuracy decrease, or spoiled motor lifespan, an elastic force direction of the damper 5, that is, the elastic force direction of the coil spring, is disposed opposite to the gravitational direction of the end-effector assembly 4. When this linear motor moves, the coil spring can provide constant force output to balance a vertical load that the third linear motor 302 bears. Benefited from following of a leaf spring of the coil spring, the design can ensure that an output elastic force of gravity compensation is in a horizontal direction of the third linear motor 302, to prevent the deformation and damage of the coil spring caused by the change in a direction of the compensated elastic force, and a force applied to the linear motor in another direction is prevented. It should be pointed out that an output force K of a constant-force clockwork spring is preset to be 2N, which is calculated by a rated thrust T of the motor, an expected maximum operating applied force A, and a born load gravity G, which can be properly adjusted based on the above parameters, and a value range of K is calculated as follows: $\left\{\begin{matrix}T + G - A \geq K \geq A + G - T ; \\ G \geq K > 0\end{matrix}\right. .$

A working principle or workflow of the embodiment: the first joint 1, the second joint 2, and the third joint 3 perform linear motion in the three axis directions, so that the end-effector assembly 4 can be driven to perform linear motion in three different directions. The first rotating motor 204 and the second rotating motor 304 provide rotational motion in two directions, finally enabling the end-effector assembly 4 to have degrees of freedom in five directions, implementing a more flexible action. When the third linear motor 302 moves, the coil spring is pulled to stretch out, and an elastic force direction of the coil spring is opposite to a load force direction of the third linear motor 302, providing force compensation for the third linear motor 302.

A beneficial effect of this embodiment: the three joints of the robot can not only implement linear motion in the three axis directions, but also implement rotary motion in at least one direction, and finally the end-effector assembly 4 can move more flexibly and reach a position of a lesion more easily. The damper 5 is disposed to prevent tremor of the third joint 3, positioning accuracy decrease, or spoiled motor lifespan of the third linear motor 302.

### Embodiment 2

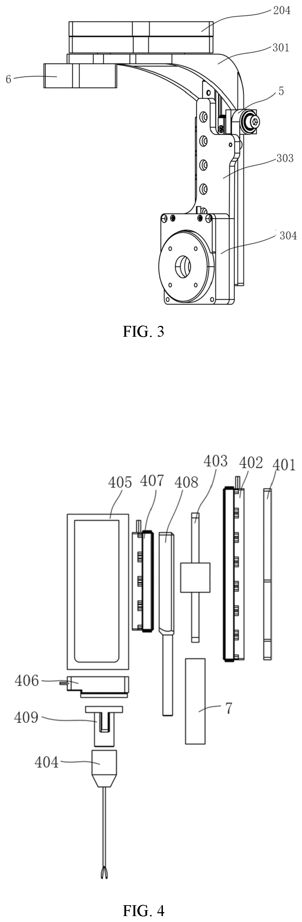

In embodiment 2 of a multi-degree-of-freedom robot, based on embodiment 1, as shown in FIG. 2 and FIG. 3, the end-effector assembly 4 and the third joint 3 are further defined.

The end-effector assembly 4 includes a connecting component 401 installed at the output end of the second rotating motor 304, a fourth linear motor 402 installed on the connecting component 401, an end installing base 403 installed at an output end of the fourth linear motor 402, and an actuator mechanism installed on the end installing base 403. The actuator mechanism includes an actuating instrument 404, an end motor base 405 installed on the end installing base 403, and an instrument rotating motor 406 installed at the bottom of the end motor base 405. The actuating instrument 404 is connected to an output end of the instrument rotating motor 406. The fourth linear motor 402 may be a linear motor, specifically a fourth linear motor.

The actuator mechanism includes an end motor base 405 installed on the end installing base 403, and an instrument rotating motor 406 installed at the bottom of the end motor base 405. The actuating instrument 404 is connected to an output end of the instrument rotating motor 406. The instrument rotating motor 406 drives the actuating instrument 404 to perform self-rotation, and rotation of the actuating instrument 404 does not cause an end of actuating instrument 404 to produce a relative displacement with a camera module 7. Therefore, the accuracy of an image collected by the camera module 7 can still be ensured. The instrument rotating motor 406 can enable the actuating instrument 404 to move more flexibly, and to facilitate the alignment of the actuating instrument 404 with a tissue lesion, and the like.

Specifically, the instrument rotating motor 406 is a hollow motor. The actuator mechanism further includes an instrument linear motor 407 installed on the end motor base 405 and a push rod 408 installed at an output end of the instrument linear motor 407. The push rod 408 passes through the end motor base 405 and the hollow motor and extends into the actuating instrument 404, and is configured to promote opening and closing of the actuating instrument 404. When the actuating instrument 404 is a surgical instrument such as microforceps and microscissors, in order to implement that the instrument is remotely controlled to be opened and closed, the push rod 408 is driven into the actuating instrument 404 by the instrument linear motor 407 to promote opening or closing of the actuating instrument 404. The instrument rotating motor 406 is connected to the actuating instrument 404 by an instrument installing base 409. The actuating instrument 404 is detachably connected to the instrument installing base 409. Because the actuating instrument 404 needs to be replaced frequently, if the actuating instrument 404 is directly connected to the instrument rotating motor 406, an output end of the instrument rotating motor 406 needs to be operated every time the actuating instrument 404 is detached, with service life easily affected. In addition, a fastener is not easily disassembled and assembled, and especially necessary disassembly and assembly tools are not provided in an operating room environment. After the instrument installing base 409 is added, the instrument installing base 409 can be connected to an output end of the instrument rotating motor 406 by a fastener, and the actuating instrument 404 only needs to be connected to the instrument installing base 409 by snap connection or threaded connection, so that the actuating instrument 404 is replaced more easily, and detachment performed on the instrument rotating motor 406 is not required.

Preferably, a counterweight block 6 is disposed on one side of the third installing plate 301 away from the third linear motor 302, and a distance between the counterweight block 6 and an axis of the first rotating motor 204 is *Dl*/2, specifically: $Dl = d \sqrt{1 + {\left(\left(l4+l0\right)cos\left(R2\right)/d\right)}^{2}}$ where *Dl* is a change value of a force arm applied to the first rotating motor 204; *l*4 is a displacement of the fourth linear motor; *l*0 is a displacement of the instrument linear motor 407; *R*2 is a rotation angle of the second rotating motor 304; and *d* is an initial value of the force arm applied to the first rotating motor 204.

A rotating axis of the first rotating motor 204 and a rotating axis of the second rotating motor 304 are disposed to be orthogonal to a central axis of an end instrument at one point, and the second rotating motor 304, the third linear motor 302, the fourth linear motor, the instrument rotating motor 406, and the instrument linear motor 407 are main loads of the first rotating motor 204, and centers of gravity of the loads are away from the first rotating motor 204. In addition, the first rotating motor 204 is a main attitude rotation mechanism and is subjected to a large load gravitational torque, and with self-rotation, and subsequent dynamic motion of the second rotating motor 304, the third linear motor 302, the fourth linear motor, the instrument rotating motor 406, and the instrument linear motor 407, the effect of the torque on the first rotating motor 204 is relatively unstable. Under the action of the counterweight block 6, a force arm applied to the first rotating motor 204 can be reduced by 2/3 cm, so that the stability of an overall structure and the smoothness of dynamic operation are ensured.

Further, the third installing plate 301 is L-shaped, a first side plate of the third installing plate 301 is connected to the output end of the first rotating motor 204, and a second side plate is configured to install the third linear motor 302. The counterweight block 6 is installed at one end of the first side plate away from the second side plate.

Remaining characteristics and a working principle in this embodiment are consistent with characteristics and a working principle in embodiment 1.

### Embodiment 3

In embodiment 3 of a multi-degree-of-freedom robot, based on embodiment 1 or embodiment 2, a difference from embodiment 1 or embodiment 2 is in that, as shown in FIG. 4, a camera module 7 is further installed on the end installing base 403, an imaging axis of the camera module 7 is parallel to an axis of the actuating instrument 404, and a focus of the camera module 7 is located on a plane perpendicular to an end of the actuating instrument 404. Because a camera module and an actuator assembly are both installed on the end installing base 403, the camera module and the actuator assembly are always in a state of moving together, so that the robot can have dynamic vision and keep a state of focusing. When the actuating instrument 404 moves in any direction, the camera module 7 can collect an image that the actuating instrument 404 is in the direction. In addition, because the actuator assembly and a motion model are separately installed on the end installing base 403, the actuator assembly and the motion model do not interfere with each other, and the camera module 7 does not block the movement of the actuator assembly. Because the camera module 7 and an end of the actuating instrument 404 are in a relatively stationary state, the camera module 7 can be always in the state of focusing to the end of the actuating instrument 404, so that the camera module 7 can take a clear image of the end of the actuating instrument 404 no matter when the actuating instrument 404 performs any motion.

Remaining characteristics and a working principle in this embodiment are consistent with characteristics and a working principle in embodiment 1 or embodiment 2.

### Embodiment 4

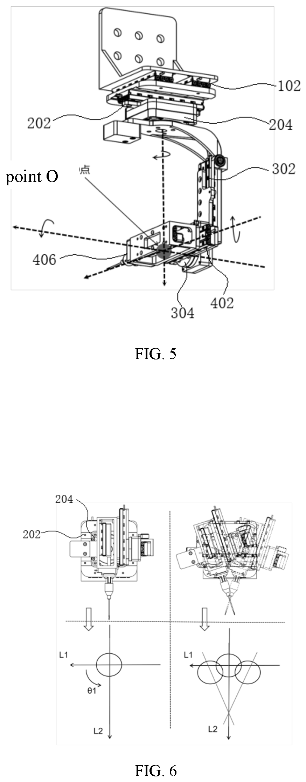

In embodiment 4 of a multi-degree-of-freedom robot, based on any of the above embodiments, a difference from any of the above embodiments is that, as shown in FIG. 5, the first linear motor 102 and the second linear motor 202 are all orthogonal to a rotating axis of the first rotating motor 204; and a motion axis of the third linear motor 302 is parallel to a rotating axis of the first rotating motor 204. A motion axis of the third linear motor 302 and a motion axis of the fourth linear motor 402 are separately orthogonal to a rotating axis of the second rotating motor 304. Rotating axes of the first rotating motor 204, the second rotating motor 304, and the instrument rotating motor 406 intersect at one point, and the point is located on an axis of the actuating instrument.

Existing surgical robots each are provided with a plurality of joints, usually with more than six joints, to ensure that the surgical robots can drive the end-effectors to perform flexible motion during operation. The surgical robot is usually designed with a structure that is provided with a plurality of rotating motors in series, to provide linear spatial freedom at an end, while a rope-driven, wire-driven, or hinge-driven wrist-like structure is used on the end-effector to provide flexible rotation and swing angle for a tool. However, the robot based on the above design has some drawbacks in the application to microsurgery. First, loads are accumulated in the plurality of rotating motors in series, so that the overall robotic arms are designed relatively large, which cannot meet narrow working space for the microsurgery in a microscope well. Second, the rope-driven, wire-driven, or hinge-driven wrist-like structure is used to provide the flexible swing angle for the tool, which may not ensure provide reliable guaranty in terms of driving accuracy, and a size (usually less than 3 mm, with an end reaching 0.05-0.1 mm) of an instrument for the microsurgery is much smaller than a size of an instrument for general surgery. Therefore, it is difficult to produce a rope-driven, wire-driven, or hinge-driven wrist-like structure with an ultra-small size. There are also some miniaturized robots such as ophthalmic surgical robots that are designed with parallelogram or linear motors in connected in parallel. Although these mechanisms can meet the needs of microsurgery in terms of size and precision, these mechanisms cannot provide flexible degrees of freedom at ends due to the lack of a wrist-like structure to provide flexible swing angles for the tool, which cannot meet the needs of complex actions such as microsurgical suture and knotting.

In this embodiment, refer to FIG. 5.
(1) Because an output axis of the first linear motor and an output axis of the second linear motor are orthogonal to the rotating axis of the first rotating motor, and a base of the second linear motor is linked to the output axis of the first linear motor, x motion and y motion respectively represented by the first linear motor and the second linear motor can be equivalently gathered at any point on the rotating axis (namely, a yaw angle of a tool) of the first rotating motor
(2) Because an output axis of the third linear motor is parallel to the rotating axis of the first rotating motor, z motion represented by the third linear motor can also equivalently coincide at any point on the rotating axis of the first rotating motor.
(3) Because the output axis of the second rotating motor is perpendicular to an output axis of the third linear motor, the output axis of the second rotating motor is equivalently perpendicular to the rotating axis of the first rotating motor. In the structure, because rotating axes of the first rotating motor and the second rotating motor intersect at one point, and the point is located on the axis of the actuating instrument, a pitch angle represented by the second rotating motor can also equivalently coincide at this point, and the point is located on the rotating axis of the first linear motor.
(4) Therefore, the point is defined as a point O. This point is on the rotating axis of the first rotating motor and intersects with the rotating axis of the second rotating motor. In the structure, because the rotating axis (roll angles) of the first rotating motor, the second rotating motor, and the instrument rotating motor intersect at the point, and the point is located on an axis of the actuating instrument, so that 6-degree-of-freedom motion at an x direction, a y direction, a z direction, a roll angle, a pitch angle, and yaw angle at the point O is transmitted.
(5) Because the fourth linear motor is installed on the rotating axis of the second rotating motor, and the output axis of the fourth linear motor is equipped with an end-tool and perpendicular to the rotating axis of the second rotating motor, the fourth linear motor can additionally provide relative axial tool motion at the point O.

Therefore, an end surgical tool has a flexibility characteristic that can reach any direction and any position within a stroke at the point O.

Beneficial effects of this embodiment are as follows: driving of the first linear motor, second linear motor, third linear motor, fourth linear motor, as well as the first rotating motor, second rotating motor, and the instrument rotating motor in the x, y, z, and tool axis directions, at the roll angle, at the pitch angle, and at the yaw angle, totaling seven degrees of freedom, are all transmitted to the point O at the central axis of an end-tool without any theoretical loss. Therefore, the end surgical tool has a characteristic of flexibly reaching any direction and any position within the stroke at the point O.

The multi-degree-of-freedom robot in this embodiment can achieve a miniaturized high-precision robot structure for working under a microscope. While a linear spatial degree of freedom is met, flexible rotation and swing angle in a virtual wrist-like structure at an end are implemented. The multi-degree-of-freedom robot provides three-axis translation and virtual fixed point control with a plurality of driving modes on a plurality of axial planes. This technology can realize a virtual wrist-like structure control with any length on the end-tool, and can be converted into a spatially fixed center point when needed to meet microsurgical scenarios such as vitreoretinal surgery that require working around a fixed wound. Therefore, the multi-degree-of-freedom robot in this embodiment can simultaneously meet the requirements of open microsurgery (incision and anastomosis of tissues, such as lymphatic, venous, and vascular tissues) and intracavitary microsurgery (such as vitreoretinal surgery performed by an ophthalmic surgical robot).

### Embodiment 5

An embodiment of a robot control method is provided, to control the multi-degree-of-freedom robot in embodiment 4.

Based on the multi-degree-of-freedom robot in embodiment 4, a first virtual wrist-like joint formed based on the driving of the first linear motor, the second linear motor, and the first rotating motor is shown in FIG. 6. A specific motion algorithm is as follows: $\left\{\begin{matrix}X = L 1 + \left(β+L4\right) sin \left(θ2\right) cos \left(θ1\right) \\ Y = L 2 + \left(β+L4\right) sin \left(θ2\right) sin \left(θ1\right) \\ α = θ 1\end{matrix}\right.$ where X and Y are orthogonal coordinate axes defined along motion directions of the first linear motor and the second linear motor respectively, equivalent to X and Y displacements at the first virtual wrist-like joint; α is a rotating axis defined along a rotation direction of the first rotating motor, equivalent to a yaw angle at the first virtual wrist-like joint; θ2 is a current angle of the second rotating motor, and θ1 is a current angle of the first rotating motor; and β is a defined virtual wrist-like joint (whose value represents a distance between the virtual wrist-like joint point and a point O when a position of the fourth linear motor is 0); and L1, L2, and L4 represent current positions of the first linear motor, the second linear motor, and the fourth linear motor respectively.

When α rotates, the first linear motor and the second linear motor perform the following motion: $\left\{\begin{matrix}{L 1}^{⋅} = \left(β+L4\right) sin \left(θ2\right) sin \left(θ1\right) {θ 1}^{⋅} \\ {L 2}^{⋅} = - \left(β+L4\right) sin \left(θ2\right) cos \left(θ1\right) {θ 1}^{⋅}\end{matrix}\right.$ where ${L 1}^{⋅}$ and ${L 2}^{⋅}$ are motion speeds executed by the first linear motor and the second linear motor respectively, and ${θ 1}^{⋅}$ is a rotation speed of the first rotating motor and α. The X and Y displacements at the first virtual wrist-like joint point can be maintained at 0, thereby implementing motion of the first virtual wrist-like joint as shown in FIG. 6.

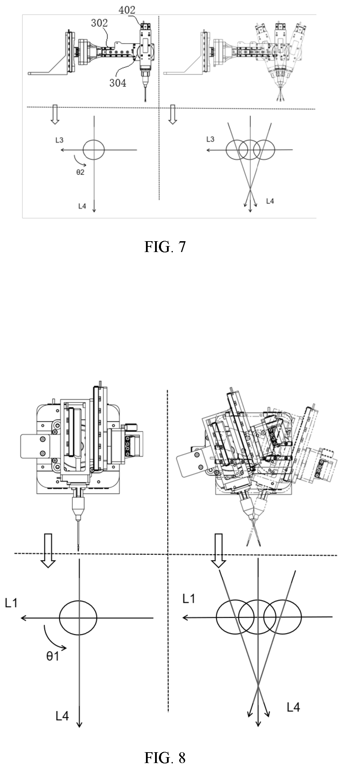

Based on the multi-degree-of-freedom robot in embodiment 4, a second virtual wrist-like joint formed based on the driving of the third linear motor, the fourth linear motor, and the second rotating motor is shown in FIG. 7. A specific motion algorithm is as follows: $\left\{\begin{matrix}X ′ = L 3 + \left(β+L4\right) cos \left(θ2\right) \\ Y ′ = \left(β+L4\right) sin \left(θ2\right) \\ α ′ = θ 2\end{matrix}\right.$

X' and Y' are coordinate axes along a motion direction of the third linear motor and the fourth linear motor swinging with the second rotating motor to a position orthogonal to the third linear motor respectively, which are equivalent to an X' displacement and a Y' displacement at the second virtual wrist-like joint point; α' is a rotating axis defined along a rotation direction of the second rotating motor, which is equivalent to a pitch angle at the second virtual wrist-like joint point; and θ2 is a current angle of the second rotating motor. β is a defined virtual wrist-like joint point (whose value represents a distance between the virtual wrist-like joint point and a point O when a position of the fourth linear motor is 0); and L3 and L4 are current positions of the third linear motor and the fourth linear motor respectively. It should be pointed out that the second virtual joint point may be the same one as the first virtual wrist-like joint point. To be specific, both the second virtual joint point and the first virtual wrist-like joint point are β, which cannot be implemented by a traditional rope-driven, wire-driven, or hinge-driven wrist-like joint due to size structure and transmission limitations.

When α' rotates, the first linear motor and the second linear motor perform the following motion: $\left\{\begin{matrix}{L 3}^{⋅} = \left(β+L4\right) sin \left(θ2\right) {θ 2}^{⋅} + \left(β+L4\right) cos \left(θ2\right) {θ 2}^{⋅} / tan \left(θ2\right) \\ {L 4}^{⋅} = - \left(β+L4\right) cos \left(θ2\right) {θ 2}^{⋅} / sin \left(θ2\right)\end{matrix}\right.$ where L3 and L4 are motion speeds executed by the third linear motor and the fourth linear motor respectively, and ${θ 2}^{⋅}$ is a rotation speed of the second rotating motor and α'. The X' displacement and Y' displacement at the second virtual wrist-like joint point can be maintained at 0, thereby implementing motion of the virtual wrist-like joint shown in FIG. 7.

A specific control method for the multi-degree-of-freedom robot based on embodiment 4 and the built first virtual wrist-like joint and second virtual wrist-like joint is as follows:
obtaining a position L1 of the first linear motor, a position L2 of the second linear motor, a position L3 of the third linear motor, a position L4 of the fourth linear motor, an angle θ1 of the first rotating motor, and an angle θ2 of the second rotating motor;
defining virtual wrist-like joint points β (assuming that two virtual wrist-like joint points use a same point on the tool), and obtaining a distance Δ between the virtual wrist-like joint point β and a control transmission point O, which is specifically: $Δ = β + L 4$

Based on the distance Δ between the virtual wrist-like joint point β and the control transmission point O, combining motion of the first linear motor, the second linear motor, the third linear motor, the fourth linear motor, the first rotating motor, and the second rotating motor into at least one linear motion generator and at least one rotating motion generator.

In this embodiment, the linear motion generator is specifically: $\left\{\begin{matrix}\left[\begin{matrix}{{L 1}^{⋅}}_{x} \\ {{L 2}^{⋅}}_{y} \\ {{L 3}^{⋅}}_{z}\end{matrix}\right] = \left[\begin{matrix}1 & 0 & 0 \\ 0 & 1 & 0 \\ 0 & 0 & 1\end{matrix}\right] \left[\begin{matrix}\dot{x} \\ \dot{y} \\ \dot{z}\end{matrix}\right] - {k}_{1} \left[\begin{matrix}cos \left(θ1\right) sin \left(θ2\right) \\ sin \left(θ1\right) sin \left(θ2\right) \\ cos \left(θ2\right)\end{matrix}\right] \left[cos\left(θ1\right)sin\left(θ2\right) sin\left(θ1\right)sin\left(θ2\right) cos\left(θ2\right)\right] \left[\begin{matrix}\dot{x} \\ \dot{y} \\ \dot{z}\end{matrix}\right] \\ {{L 4}^{⋅}}_{l} = {k}_{1} \left[cos\left(θ1\right)sin\left(θ2\right) sin\left(θ1\right)sin\left(θ2\right) cos\left(θ2\right)\right] \left[\begin{matrix}\dot{x} \\ \dot{y} \\ \dot{z}\end{matrix}\right]\end{matrix}\right.$ where *k*₁ is a parameter factor ranging from 0 to 1. When *k*₁ = 0, only the first linear motor, the second linear motor, and the third linear motor are used to complete linear movement. Benefited from the axial motion of a tool of the fourth linear motor intersecting with the first linear motor, the second linear motor, and the third linear motor at the point O, when *k*₁ > 0, due to a flexibility characteristic of an end surgical tool, at the point O, that any direction and position is reachable within a stroke (namely, output axes of the fourth linear motor, the first linear motor, the second linear motor, and the third linear motor intersect at the point O), the fourth linear motor compensates for the linear movement to some extent, thereby reducing a motion stroke of other linear motors.

In this embodiment, three rotating motion generators are provided, namely, a first virtual rotating generator, a second virtual rotating generator, and a rotating generator.

The first virtual rotating generator is specifically: $\left[\begin{matrix}{{L 1}_{a}}^{⋅} \\ {{L 2}_{a}}^{⋅} \\ {θ 1}^{⋅}\end{matrix}\right] = {\left[\begin{matrix}1 & 0 & - Δsin \left(θ1\right) sin \left(θ2\right) \\ 0 & 1 & Δcos \left(θ1\right) sin \left(θ2\right) \\ 0 & 0 & 1\end{matrix}\right]}^{+} \left[\begin{matrix}0 \\ 0 \\ \dot{a}\end{matrix}\right]$

The second virtual rotating generator is specifically: $\left[\begin{matrix}{L {3}_{b}}^{⋅} \\ {{L 4}_{b}}^{⋅} \\ {θ 2}^{⋅}\end{matrix}\right] = {\left[\begin{matrix}1 & cos \left(θ2\right) & - Δsin \left(θ2\right) \\ 0 & sin \left(θ2\right) & Δcos \left(θ2\right) \\ 0 & 0 & 1\end{matrix}\right]}^{+} \left[\begin{matrix}0 \\ 0 \\ \dot{b}\end{matrix}\right]$

The rotating generator is specifically: ${θ 3}^{⋅} = \dot{c}$
where Δ represents the distance between the virtual wrist-like joint point β and the control transmission point O. It should be pointed out that for ease of description herein, it is assumed that a same point β on the tool is used for the first virtual rotating generator and the second virtual rotating generator, that is, a same parameter Δ is used for the two virtual rotating generators. In practical application, the second virtual rotating generator can be defined as a virtual wrist-like joint point β1 that is completely different from the first virtual rotating generator, and a form of the second virtual rotating generator remains unchanged, with only a parameter Δ changed (Δ1 = β1 + L4).
where L4 is a current position of the fourth linear motor; θ1 and θ2 are current angles of the first rotating motor and the second rotating motor respectively, ${{L 1}^{⋅}}_{x} , {{L 2}^{⋅}}_{y} , {{L 3}^{⋅}}_{z}$, and ${{L 4}^{⋅}}_{l}$ represent operating speeds of the first linear motor, the second linear motor, the third linear motor, and the fourth linear motor respectively for executing the linear motion generator; ${{L 1}^{⋅}}_{a}$ and ${{L 2}^{⋅}}_{a}$ are operating speeds of the first linear motor and the second linear motor respectively for executing the first virtual rotating generator; and ${{L 3}^{⋅}}_{b}$ and ${{L 4}^{⋅}}_{b}$ are operating speeds of the third linear motor and the fourth linear motor respectively for executing the second virtual rotating generator.
x, y, and x are positions of the actuating instrument on a coordinate axis; ẋ, ẏ, ż is a linear displacement speed of the actuating instrument in an expected instruction; a and b are rotation angles of the actuating instrument; ȧ, ḃ, ċ is a rotational speed of the actuating instrument in the expected instruction; ${θ 2}^{⋅}$ is a rotational speed of the first rotating motor; ${θ 2}^{⋅}$ is a rotational speed of the second rotating motor; and ${θ 3}^{⋅}$ is a rotational speed of the instrument rotating motor.

The expected instruction [ẋ, ẏ, ż, ȧ, b, ċ, ] is input into the linear motion generator and the rotating motion generator, and outputs are accumulated to obtain motion outputs of the first linear motor, the second linear motor, the third linear motor, the fourth linear motor, the first rotating motor, and the second rotating motor.

In this embodiment, based on the multi-degree-of-freedom robot in embodiment 4, a same angle input ȧ and ḃ is selected as follows for the first virtual rotating generator and the second virtual rotating generator:
When the first rotating motor is at 90°, as shown in FIG. 8, the first virtual rotating generator can be combined by the first linear motor and the fourth linear motor: $\left[\begin{matrix}{{L 1}^{⋅}}_{a} \\ {{L 4}^{⋅}}_{a} \\ {θ 1}^{⋅}\end{matrix}\right] = {\left[\begin{matrix}1 & sin \left(θ2\right) cos \left(θ1\right) & - Δsin \left(θ2\right) sin \left(θ1\right) \\ 0 & sin \left(θ2\right) sin \left(θ1\right) & Δsin \left(θ2\right) cos \left(θ1\right) \\ 0 & 0 & 1\end{matrix}\right]}^{+} \left[\begin{matrix}0 \\ 0 \\ \dot{a}\end{matrix}\right]$

Similarly, when the first rotating motor is at 0°, the first virtual rotating generator can be combined by the second linear motor and the fourth linear motor: $\left[\begin{matrix}{{L 2}^{⋅}}_{a} \\ {{L 4}^{⋅}}_{a} \\ {θ 1}^{⋅}\end{matrix}\right] = {\left[\begin{matrix}1 & sin \left(θ2\right) cos \left(θ1\right) & - Δsin \left(θ2\right) sin \left(θ1\right) \\ 0 & sin \left(θ2\right) sin \left(θ1\right) & Δsin \left(θ2\right) cos \left(θ1\right) \\ 0 & 0 & 1\end{matrix}\right]}^{+} \left[\begin{matrix}0 \\ 0 \\ \dot{a}\end{matrix}\right]$

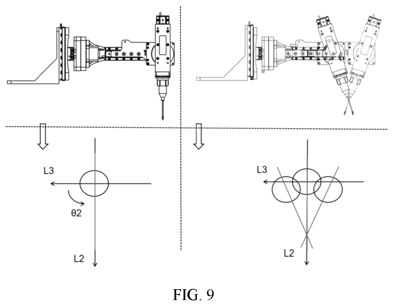

When the first rotating motor is at 90°, as shown in FIG. 9, the second virtual rotating generator can be combined by the second linear motor and the third linear motor: $\left[\begin{matrix}{{L 2}_{a}}^{⋅} \\ {{L 3}_{a}}^{⋅} \\ {θ 1}^{⋅}\end{matrix}\right] = {\left[\begin{matrix}1 & 0 & - Δsin \left(θ2\right) \\ 0 & 1 & Δcos \left(θ2\right) \\ 0 & 0 & 1\end{matrix}\right]}^{+} \left[\begin{matrix}0 \\ 0 \\ \dot{b}\end{matrix}\right]$

Similarly, when the first rotating motor is at 0°, the second virtual rotating generator can be combined by the first linear motor and the third linear motor: $\left[\begin{matrix}{{L 1}_{a}}^{⋅} \\ {{L 3}_{a}}^{⋅} \\ {θ 1}^{⋅}\end{matrix}\right] = {\left[\begin{matrix}1 & 0 & - Δsin \left(θ2\right) \\ 0 & 1 & Δcos \left(θ2\right) \\ 0 & 0 & 1\end{matrix}\right]}^{+} \left[\begin{matrix}0 \\ 0 \\ \dot{b}\end{matrix}\right]$

Beneficial effects of the embodiment: based on the multi-degree-of-freedom robot in embodiment 4, in a set position relationship among the first linear motor, the second linear motor, the third linear motor, the fourth linear motor, the first rotating motor, the second rotating motor, and the instrument rotating motor of the robot, a virtual wrist-like structure can be formed through algorithm control, which is exempted from a rope-driven mechanical structure or the like, and may not rely on the motion of the mechanical structure. Compared with traditional mechanical wrist-like joints, the multi-degree-of-freedom robot ensures higher precision, high operational reliability (preventing rope-driven mechanical fatigue), and a lower production difficulty. Similarly, due to motion flexibility of the multi-degree-of-freedom robot in embodiment 4, a variety of remote center motion of wrist-like joints can be performed in a plurality of spatial axial planes.

In addition, the control method based on this embodiment and the corresponding multi-degree-of-freedom robot shield the linear motion generator, motion of the first virtual rotating generator, the second virtual rotating generator, the rotating generator, and the fourth linear motor is used, and Δ is set to β, namely, a virtual rotating fixed point β is a fixed position, with a distance from the point O, that is set along the tool direction, so that intracavity RCM motion around a wound or trocar fixed point can be implemented without following the movement of the tool (axial motion of the tool in a cavity can be directly provided by the fourth linear motor).

Obviously, the above embodiments of the present invention are only examples for clearly describing the present invention and are not a limitation of the embodiments of the present invention. For those skilled in the art, other different forms of changes or modifications can be made based on the above description. All implementations are not to be listed herein. Any modifications, equivalent substitutions, improvements, and the like made within the spirit and principle of the present invention should be included within the protection scope of the claims of the present invention.

## Claims

1. A multi-degree-of-freedom robot, comprising a first joint (1), a second joint (2), a third joint (3), and an end-effector assembly (4) that are connected sequentially, **characterized in that**, the first joint (1), the second joint (2), the third joint (3), and the end-effector assembly (4) each comprise at least one linear motor and an installing plate for installing the linear motor; and at least one of the first joint (1), the second joint (2), and the third joint (3) further comprises a rotating motor.

2. The multi-degree-of-freedom robot according to claim 1, **characterized in that**, a damper (5) is installed on the installing plate corresponding to the linear motor whose output axis is parallel to a gravitational direction of the end-effector assembly (4), and an output end of the damper (5) is connected to an output end of the linear motor.

3. The multi-degree-of-freedom robot according to claim 1, **characterized in that**, the first joint (1) comprises a first installing plate (101) and a first linear motor (102) connected to the first installing plate (101); the second joint (2) comprises a second installing plate (201) connected to an output end of the first linear motor (102), a second linear motor (202) connected to the second installing plate (201), a first installing component (203) connected to an output end of the second linear motor (202), and a first rotating motor (204) installed on the first installing component (203); and the third joint (3) comprises a third installing plate (301) connected to an output end of the first rotating motor (204), a third linear motor (302) connected to the third installing plate (301), a second installing component (303) connected to an output end of the third linear motor (302), and a second rotating motor (304) connected to the second installing component (303); and a connecting component (401) is connected to an output end of the second rotating motor (304).

4. The multi-degree-of-freedom robot according to claim 3, **characterized in that**, an output axis of the second linear motor (202) is perpendicular to an output axis of the first linear motor (102), and an output axis of the third linear motor (302) is perpendicular to both the output axis of the second linear motor (202) and the output axis of the first linear motor (102); and a rotating axis of the first rotating motor (204) is perpendicular to the output axis of the second linear motor (202), a rotating axis of the second rotating motor (304) is perpendicular to the output axis of the third linear motor (302), and the output axis of the third linear motor (302) is parallel to a gravitational direction of the end-effector assembly (4).

5. The multi-degree-of-freedom robot according to claim 4, **characterized in that**, the end-effector assembly (4) comprises the connecting component (401) installed at the output end of the second rotating motor (304), a fourth linear motor (402) installed on the connecting component (401), an end installing base (403) installed at an output end of the fourth linear motor (402), and an actuator mechanism installed on the end installing base (403); and the actuator mechanism comprises an actuating instrument (404), an end motor base (405) installed on the end installing base (403), and an instrument rotating motor (406) installed at the bottom of the end motor base (405); and the actuating instrument (404) is connected to an output end of the instrument rotating motor (406).

6. The multi-degree-of-freedom robot according to claim 5, **characterized in that**, a counterweight block (6) is disposed on one side of the third installing plate (301) away from the third linear motor (302), and a distance between the counterweight block (6) and an axis of the first rotating motor (204) is *Dl*/2, specifically as follows: $Dl = d \sqrt{1 + {\left(\left(l4+l0\right)cos\left(R2\right)/d\right)}^{2}}$ wherein *Dl* is a change value of a force arm applied to the first rotating motor (204); *l*4 is a displacement of the fourth linear motor; *l*0 is a displacement of the instrument linear motor (407); *R*2 is a rotation angle of the second rotating motor (304); and *d* is an initial value of the force arm applied to the first rotating motor (204).

7. The multi-degree-of-freedom robot according to claim 6, **characterized in that**, the third installing plate (301) is L-shaped, a first side plate of the third installing plate (301) is connected to the output end of the first rotating motor (204), and a second side plate is configured to install the third linear motor (302); and the counterweight block (6) is installed at one end of the first side plate away from the second side plate.

8. The multi-degree-of-freedom robot according to claim 5, **characterized in that**, the instrument rotating motor (406) is a hollow motor; the actuator mechanism further comprises an instrument linear motor (407) installed on the end motor base (405) and a push rod (408) installed at an output end of the instrument linear motor (407); the push rod (408) passes through the end motor base (405) and the hollow motor and extends into the actuating instrument (404) and is configured to promote opening and closing of the actuating instrument (404); the instrument rotating motor (406) is connected to the actuating instrument (404) through an instrument installing base; and the actuating instrument (404) is detachably connected to the instrument installing base (409).

9. The multi-degree-of-freedom robot according to claim 5, **characterized in that**, rotating axes of the first linear motor (102), the second linear motor (202), and the first rotating motor (204) are orthogonal in pairs; a motion axis of the third linear motor (302) is parallel to a rotating axis of the first rotating motor (204); motion axes of the third linear motor (302) and the fourth linear motor (402) are orthogonal to a rotating axis of the second rotating motor (304); and rotating axes of the first rotating motor (204), the second rotating motor (304), and the instrument rotating motor (406) intersect at one point, and the point is located on an axis of the actuating instrument.

10. A robot control method, **characterized in that**, the robot control method is used to control the multi-degree-of-freedom robot according to claim 9 and comprises:
obtaining a position L1 of the first linear motor, a position L2 of the second linear motor, a position L3 of the third linear motor, a position L4 of the fourth linear motor, an angle θ1 of the first rotating motor, and an angle θ2 of the second rotating motor;
defining a virtual wrist-like joint point β, and obtaining a distance between the virtual wrist-like joint point β and a control transmission point O;
based on the distance between the virtual wrist-like joint point β and the control transmission point O, combining motion of the first linear motor, the second linear motor, the third linear motor, the fourth linear motor, the first rotating motor, and the second rotating motor into at least one linear motion generator and at least one rotating motion generator; and
inputting an expected instruction into the linear motion generator and the rotating motion generator, and accumulating outputs to obtain motion outputs of the first linear motor, the second linear motor, the third linear motor, the fourth linear motor, the first rotating motor, and the second rotating motor.

11. The robot control method according to claim 10, **characterized in that**, the distance between the virtual wrist-like joint point β and the control transmission point O is specifically: $Δ = β + L 4$
the linear motion generator is specifically: $\left\{\begin{matrix}\left[\begin{matrix}{{L 1}^{⋅}}_{x} \\ {{L 2}^{⋅}}_{y} \\ {{L 3}^{⋅}}_{z}\end{matrix}\right] = \left[\begin{matrix}1 & 0 & 0 \\ 0 & 1 & 0 \\ 0 & 0 & 1\end{matrix}\right] \left[\begin{matrix}\dot{x} \\ \dot{y} \\ \dot{z}\end{matrix}\right] - {k}_{1} \left[\begin{matrix}cos \left(θ1\right) sin \left(θ2\right) \\ sin \left(θ1\right) sin \left(θ2\right) \\ cos \left(θ2\right)\end{matrix}\right] \left[cos\left(θ1\right)sin\left(θ2\right) sin\left(θ1\right)sin\left(θ2\right) cos\left(θ2\right)\right] \left[\begin{matrix}\dot{x} \\ \dot{y} \\ \dot{z}\end{matrix}\right] \\ {{L 4}^{⋅}}_{l} = {k}_{1} \left[cos\left(θ1\right)sin\left(θ2\right) sin\left(θ1\right)sin\left(θ2\right) cos\left(θ2\right)\right] \left[\begin{matrix}\dot{x} \\ \dot{y} \\ \dot{z}\end{matrix}\right]\end{matrix}\right.$
three rotating motion generators are provided, the three rotating motion generators are a first virtual rotating generator, a second virtual rotating generator, and a rotating generator respectively;
the first virtual rotating generator is specifically: $\left[\begin{matrix}{L {1}_{a}}^{⋅} \\ {{L 2}_{a}}^{⋅} \\ {θ 1}^{⋅}\end{matrix}\right] = {\left[\begin{matrix}1 & 0 & - Δsin \left(θ1\right) sin \left(θ2\right) \\ 0 & 1 & Δcos \left(θ1\right) sin \left(θ2\right) \\ 0 & 0 & 1\end{matrix}\right]}^{+} \left[\begin{matrix}0 \\ 0 \\ \dot{a}\end{matrix}\right]$
the second virtual rotating generator is specifically: $\left[\begin{matrix}{L {3}_{b}}^{⋅} \\ {{L 4}_{b}}^{⋅} \\ {θ 2}^{⋅}\end{matrix}\right] = {\left[\begin{matrix}1 & cos \left(θ2\right) & - Δsin \left(θ2\right) \\ 0 & sin \left(θ2\right) & Δcos \left(θ2\right) \\ 0 & 0 & 1\end{matrix}\right]}^{+} \left[\begin{matrix}0 \\ 0 \\ \dot{b}\end{matrix}\right]$
the rotating generator is specifically: ${θ 3}^{⋅} = \dot{c}$
wherein *k*₁ is a parameter factor ranging from 0 to 1; and Δ is the distance between the virtual wrist-like joint point β and the control transmission point O; L4 is a current position of the fourth linear motor; θ1 and θ2 are current angles of the first rotating motor and the second rotating motor respectively, ${{L 1}^{⋅}}_{x} , {{L 2}^{⋅}}_{y} , {{L 3}^{⋅}}_{z}$, and ${{L 4}^{⋅}}_{l}$ represent operating speeds of the first linear motor, the second linear motor, the third linear motor, and the fourth linear motor respectively for executing the linear motion generator; ${{L 1}^{⋅}}_{a}$ and ${{L 2}^{⋅}}_{a}$ are operating speeds of the first linear motor and the second linear motor respectively for executing the first virtual rotating generator; and ${{L 3}^{⋅}}_{b}$ and ${{L 4}^{⋅}}_{b}$ are operating speeds of the third linear motor and the fourth linear motor respectively for executing the second virtual rotating generator; and
x, y and x are positions of the actuating instrument on a coordinate axis; ẋ, ẏ, ż is a linear displacement speed of the actuating instrument in an expected instruction; a and b are rotation angles of the actuating instrument; ȧ, ḃ, ċ is a rotational speed of the actuating instrument in the expected instruction; ${θ 2}^{⋅}$ is a rotational speed of the first rotating motor; ${θ 2}^{⋅}$ is a rotational speed of the second rotating motor; and ${θ 3}^{⋅}$ is a rotational speed of the instrument rotating motor.
